# EUROPEAN PATENT APPLICATION

(11) **EP 2 687 844 A2**
(43) Date of publication of application: **22.01.2014**
(21) Application number: 12382543.2
(22) Date of filing: 28.12.2012
(51) Int. Cl.: G01N 33/00

(54) **Air quality monitoring device**

(30) Priority: 16.07.2012 ES 201231110
(71) Applicant: Fundacion Cartif, 47151 Boecillo (ES); Sociedad de Estudios P y G, S.A., 28001 Madrid (ES)
(72) Inventor: Hidalgo Barrio, María Dolores, 47151 BOECILLO (Valladolid) (ES); Gómez Rincón, Marta, 47151 BOECILLO (Valladolid) (ES); Sastre, García, Emilia, 47151 BOECILLO (Valladolid) (ES); García-Blanch de Benito, Francisco, 47151 BOECILLO (Valladolid) (ES)
(74) Representative: Capitán García, Maria Nuria

(57) **Abstract**

The present invention relates to a device for monitoring air quality for the control of pollutants in urban air, so that the pollution levels, mainly associated with traffic, can be reduced thanks to this information.

## Description

### TECHNICAL FIELD OF THE INVENTION

The present invention relates to a device for monitoring air quality for the control of pollutants in urban air, so that this information can help reduce pollution levels, which are mainly related to road traffic.

### BACKGROUND OF THE INVENTION

Polluted air in cities is a very important problem. The main source of pollution is transportation, vehicles circulating in the city and gases they emit into the atmosphere. In addition, this situation is worsened in the city downtown, where the narrowness of some streets makes it very difficult the dispersion of pollutants.

Pollution in cities can provoke respiratory illnesses to people living there. It is therefore very important to measure urban pollution levels, to know at all times the concentration of pollutants in air to protect citizens and to control traffic pollution.

Nowadays, main European cities measure pollution levels by means of fixed stations located within a few points of urban geography or estimate these by using meteorological models for predicting future pollution.

It would be highly advisable to develop new air monitoring devices to help control air quality in cities.

### DESCRIPTION OF THE INVENTION

Present invention is established and characterised in the independent claims, while the dependent claims describe its other characteristics.

The air quality monitoring device of the invention comprises a protective case where the different elements needed for measuring pollutants in the air, processing and transmission of the data collected besides an energy source for the device operation are integrated. Casing plus the elements which are supported on it, form the head of the device. The head is inserted on the top of a pole which will be co-located on the streets where air quality is to be measured.

The device of the invention is compact, functional, durable, low cost, energy autonomous, with ability to gather multiple sensors and anti-vandalism protection.

In view of the above statement, the present invention relates to a device for monitoring air quality that comprises a head which in turn comprises a protective case housing a power supply system, sensors for measuring air pollutants, a data processing unit and a data transmission unit. The head is situated inside of a pole, so that the upper side of the case is levelled at the upper end thereof; the post shows at least one opening for the passage of the air to be analysed.

The pole where is housed the head described in the invention ensures the integrity of the protected items, so they do not degrade and remain functional in spite of being exposed to atmospheric elements and/or possible vandalism in a public road.

### BRIEF DESCRIPTION OF FIGURES

The present descriptive report is complemented with a set of illustrative figures of the preferred embodiment, but never restrictive of the invention.

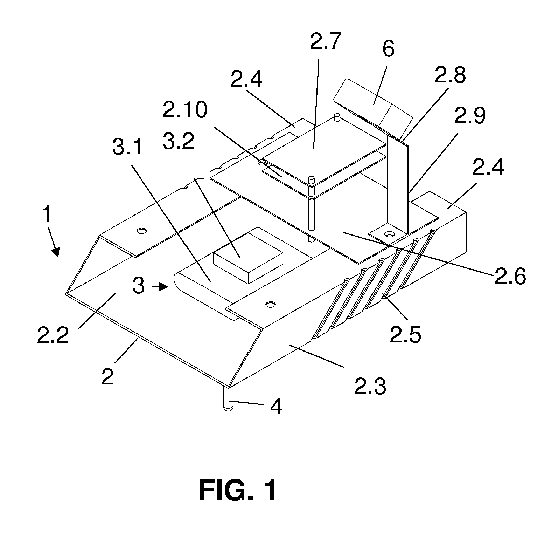

Figure 1 represents a perspective view of the invention head.
Figure 2 represents an exploded view of the invention device.

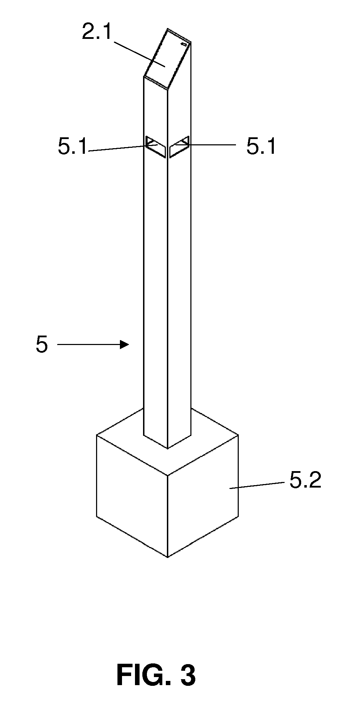

Figure 3 represents a perspective view of the invention device.

### DETAILED EXPLANATION OF A PREFERRED EMBODIMENT OF THE INVENTION

In view of the statements above, the present invention relates to a device for monitoring air quality comprising: a head (1) which in turn gathers a protective case (2) where a power supply system (3), sensors for measuring air quality, a data processing unit (for data collected by the sensors (not represented)) and a data transmission unit (4) are integrated, where the head (1) is disposed inside a pole (5), so that the upper side of the case (2.1) is levelled at the upper end thereof; the pole (5) shows at least one opening (5.1) as inlet to the air to be analysed.

The protective case (2) is configured to provide support, in the smallest possible space, to the set of elements necessary for the monitoring air quality.

In a particular picture, the case (2) shows a "C" profile. "C" profile defines an upper face (2.1), a lower face (2.2), two side faces (2.3) and two fins (2.4) below to the side faces (2.3).

The case (2) can show grids (2.5) in its lateral sides (2.3) for passage of analysed air.

The case (2) has also a support board (2.6) whose ends are attached to the fins (2.4) in order to provide a support point to the main electronic board (2.10), to the sensors electronic board (2.7) which is attached and parallel to the main electronic board (2.10) and to the leaning sensors board (2.8).

Particularly, the data processing unit is placed in the main electronic board (2.10) onto the support board (2.6) but not attached to it, in the protective case (2), and the sensors for measuring air quality are situated on the sensors electronic board (2.7) attached and parallel to the main electronic board (2.10) in the protection case (2) or in a leaning sensors board (2.8) after a bottom (2.9) whenever the airflow is needed perpendicular to the sensor (6).

Preferably, sensors (6) that need to be perpendicular to the airflow, for example particulate sensors, are situated onto a leaning sensors board (2.8) located after a bottom (2.9), with a slope between 40º and 50º so that sensors are detached from the case (2) in order to the sampling is optimally fulfilled. Preferably the inclination is mostly 45º.

The data processing unit includes a microcontroller where the code that managing communications and sensors is implemented.

The selected sensors allow for the measurement of atmospheric indicators most relevant to health. In particular, the sensors are embodied in a printed circuit board that holds the pollutant measurement and traffic flow sensors, and forwards measurements to the data processing unit. In all cases, they are used sensors embedded to ensure a compact set. Examples of sensors are: CO, O₃, NO₂, particulates, noise, temperature and humidity.

As already said, the configuration of the protective case (2) allows the arrangement of different elements in the smallest possible space. Thus, in the protective case (2) the power supply system (3) which includes an energy source (3.1) and an energy charge controller is located (3.2). Specifically, the energy source (3.1) is a lithium ion battery. The lithium ion battery (3.1) is recharged with solar radiation captured by a photovoltaic panel (not represented). The recharge of the battery (3.1) and the photovoltaic panel are controlled by a charge controller (3.2). Specifically, the photovoltaic panel is situated onto the top side (2.1) of the protective case (2) and the battery (3.1) and the charge controller (3.2) at the bottom side (2.2) the controller being placed over the battery (3.1).

The integration of a charge controller (3.2) with the photovoltaic panel provides full energy autonomy to the device of the invention. The charge controller (3.2) links the photovoltaic panels with the batteries (3.1). The charge controller (3.2) is the device responsible for protecting the battery (3.1) against overloads and bottom discharges. The charge controller (3.2) constantly monitors the charge status of the batteries and regulates charge intensity to extend their lifetime; the charge controller has the function that batteries are not overloaded or unloaded longer than needed.

Besides, the charge controller prevents the overloading of the battery (3.1) by the photovoltaic panel through voltage regulation, keeping it always below a certain limit. The battery (3.1) will indicate that it cannot continue accepting power as soon as it exceeds a certain load limit. The charge controller (3.2) lowers the current as is reaching this limit, in order to decrease the amount of charging current of the battery (3.1). The charge controller (3.2) is capable of measuring the status of battery voltage (3.1) and when the battery (3.1) is fully loaded, the controller (3.2) either stops or slows down the amount of current flowing into the battery (3.1) from the photovoltaic panel. The charge controller (3.2) has to fit the voltage of the photovoltaic panel and it must be capable to handle the peak current from the panel, which flows through the controller (3.2) at a certain time. The charge controller (3.2) has a microprocessor for properly managing the photovoltaic system. Its programming allows control be able to automatically adapt to different situations.

The data transmission unit is embodied into a communication antenna (4) that is located on the top side (2.1) of the protective case (2). Communication is undertaken with the support of a wireless modem.

The head assembly (1) is inserted into the top of a pole (5) so that the upper side (2.1) of the case is levelled with the top of the pole section (5). The pole (5) must have at least one opening (5.1) as inlet to the air to be analysed. In the present case, it has four openings (5.1).

The pole has the opening or openings (5.1) at the height of a human nose in order to carry out a sampling as similar as possible to air breathed by pedestrians, and it also provides the system with anti-vandalism protection.

Sampling is carried out as follows, the air enters through the opening or openings (5.1) of the pole (5), which is located at the height of a human nose and circulates to the top of it (5), where the sensors are situated, and exits through the grids (2.5) of the case (2).

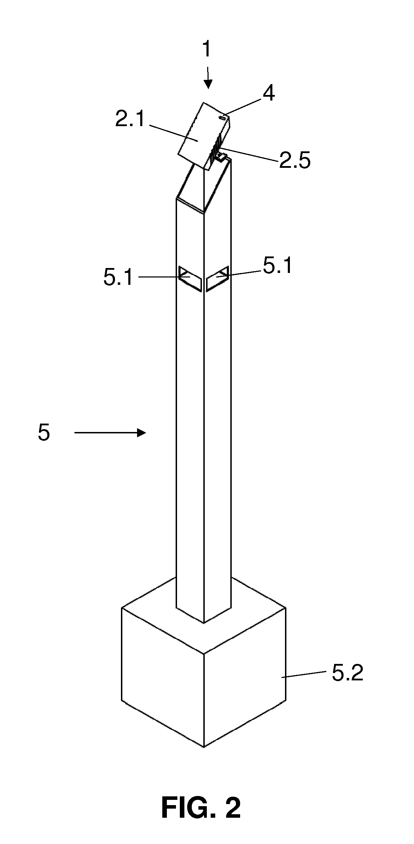

The head (1) is placed inside the pole, inclined 45º, so the larger elements, sensors and the data processing unit are situated at the bottom (avoiding encounter the wall of the pole) and the battery (3.1) and the charge controller (3.2) are situated on the top, as shown in FIG. 2.

The monitoring device of the invention becomes really effective when a network of devices covering the whole surface to monitor is spread across a city. With this configuration, each of the devices can communicate with each other or with an ending device operating as concentrator of the network data.

## Claims

1. Air quality monitoring device which comprises: a head (1) which in turn comprises a protective case (2) where a power supply system (3), sensors for measuring air quality, a data processing unit (for the data collected by the sensors (not represented)) and a data transmission unit (4) are integrated, where the head (1) is situated inside a pole (5), so that the upper side of the case (2.1) is levelled to its upper limit; the pole (5) has at least one opening (5.1) as inlet to the air to be analysed.

2. Air monitoring device according to claim 1 where the case (2) shows a "C" profile that defines an upper face (2.1), a lower face (2.2), two side faces (2.3) and two fins (2.4) below to the side faces (2.3).

3. Air monitoring device according to claim 1-2 where the case (2) shows in its lateral sides (2.3), grids (2.5) for the passage of the analysed air.

4. Air monitoring device according to claim 1-3 where the case (2) has a support board (2.6) whose ends are attached to the fins (2.4) in order to provide a support point to the main electronic board (2.10), to the sensors electronic board (2.7) which is attached and parallel to the main electronic board (2.10) and to the leaning sensors board (2.8).

5. Air monitoring device according to claim 4 where the inclined sensors board (2.8) is located after a bottom (2.9), with a slope between 40º and 50º.

6. Air monitoring device according to claim 1-5 where the power supply system (3) includes an energy source (3.1) and an energy charge controller (3.2).

7. Air monitoring device according to claim 6 where the energy source (3.1) is a lithium ion battery, the lithium ion battery (3.1) is recharged with solar radiation captured by a photovoltaic panel and the recharge of the battery (3.1) and the photovoltaic panel are controlled by a charge controller (3.2).

8. Air monitoring device according to claim 1-7 where the head (1) is inserted into the top of a pole (5) so that the upper side (2.1) of the case is levelled with the top of the pole section (5), pole which has at least one opening (5.1) for the inlet of air to be analysed.
